# EUROPEAN PATENT SPECIFICATION

(11) **EP 1 272 652 B1**
(45) Date of publication and mention of the grant of the patent: **06.10.2004**
(21) Application number: 01919668.2
(22) Date of filing: 12.04.2001
(51) Int. Cl.: C12N 15/869, A61K 48/00, C12N 15/86

(54) **HERPES VIRUSES FOR IMMUNE MODULATION**
HERPESVIREN FÜR DIE MODULATION EINER IMMUNANTWORT
VIRUS DE L'HERPES DESTINES A L'IMMUNOMODULATION

(30) Priority: 12.04.2000 GB 0009079
(43) Date of publication of application: 08.01.2003
(73) Proprietor: BioVex Limited, Oxford OX14 4RX (GB)
(72) Inventor: COFFIN, Robert, Stuart The Windeyer Institute, London W1P 6DB (GB)
(74) Representative: Woods, Geoffrey Corlett
(86) International application number: PCT/GB2001/001666
(87) International publication number: WO 2001/077358

(56) References cited:
- WO-A-00/08191
- WO-A-97/13866
- WO-A-98/04726

## Description

### FIELD OF THE INVENTION

The present invention relates to attenuated herpes simplex viruses capable of efficiently infecting dendritic cells. It also relates to the use of such viruses in immunotherapy approaches to the treatment of disease.

### BACKGROUND TO THE INVENTION

Dendritic cells (DCs) are the most potent antigen presenting cells and are efficient at inducing responses even to antigens to which the immune system has become tolerant. Thus for tumour immunotherapy, in which an immune response is raised against a tumour, the use of DCs may be ideal if they were made to present tumour specific antigens. DCs might also be used to present antigens derived from infectious agents, such as bacteria, viruses or parasites, providing protective or therapeutic vaccines for such diseases. However effective transfer of antigens into DCs for any of these targets has proved the greatest problem with this approach.

To provide a realistic chance of generating a therapeutic immune response against a tumour antigen or other disease related antigen, several conditions have to be met. Firstly, it is necessary to identify molecules whose expression is tumour or disease specific (or at least selective), and which can therefore serve as the target for an immune response. This task has proved very difficult for the majority of common tumours, but is solved in for example the case of cervical cancer by the presence, in most cases, of the viral oncogenes E6 and E7, and for other tumours, good candidate antigens are beginning to be identified. For example the MUC-1 gene product is over expressed in a number of tumours, including 90% of ovarian cancers. Various other tumour associated antigens have also been identified, any of which might be used in an immunotherapy treatment of cancer. Further tumor associated antigens will no doubt continue to be discovered over time. Secondly, following the identification of the antigen/antigens, it is necessary to deliver the antigens in an immunogenic form to the immune system. To generate the cellular immune response critical for tumour rejection, this means the proteins must either be delivered inside the cytoplasm of a host cell (a difficult task for high molecular weight protein antigens) or synthesized by the host cells themselves after gene delivery or DNA immunisation. Viral vectors which have been considered for this purpose include vaccinia, adenoviruses, or retroviruses.

The cell-type which is now widely recognised as providing the optimal immune stimulus is the dendritic cell (DC; see for example Girolomoni and Ricciardi-Castagnoli, 1997). Indeed the DC appears to be the only cell-type capable of stimulating a primary immune response *in vivo*, and moreover has even been shown to be capable of breaking established tolerance in certain circumstances. A number of groups are exploring the use of DCs in autologous adoptive immunotherapy protocols to stimulate immune responses against tumours in the hope that they may show a therapeutic effect. Such protocols involve culture and/or enrichment of DCs from peripheral blood, *in vitro* loading of DCs with antigen and reintroduction of the DCs to the patient or direct *in vivo* loading of DCs with antigen. However this approach has been hampered by the absence of efficient means by which to load these cells with antigens. Recent work has however shown that presentation of antigens by peptide pulsed DCs has produced anti-tumour responses *in vivo* (Celluzzi *et al*., 1996; *Zitvogel et al*., 1996). As regard to viral vectors, retroviruses do not give high efficiency gene delivery to dendritic cells (Reeves *et al*., 1996; Aicher *et al*., 1997), and in our hands, unlike work reported by others (Arthur *et al*., 1997), adenoviruses only give low efficiency gene delivery.

We have previously tested and reported that herpes simplex viruses (HSV) can efficiently infect and deliver genes to dendritic cells (Coffin *et al*., 1998; WO 00/08191). HSV has a number of advantages over other vector systems for this purpose, in that it can efficiently infect a wide variety of cell-types (including some very hard to infect with other vector systems e.g. Dilloo *et al*., 1997; Coffin *et al*., 1998), is easy to manipulate, and can accept large DNA insertions allowing the expression of multiple genes (reviewed by Coffin and Latchman 1996). Delivery of multiple antigens to dendritic cells *ex vivo* followed by re-introduction into the body or direct administration of antigens to dendritic cells *in vivo* may be particularly promising approaches to the treatment of some cancers and infectious diseases.

WO 00/08191 teaches that wild type herpes simplex viruses prevent antigen processing occurring in infected dendritic cells and that herpes viruses that either lack both functional UL43 and vhs genes or contain mutations that minimise immediate early gene expression are capable of efficiently infecting dendritic cells without preventing antigen processing occurring in the infected cells.

### SUMMARY OF THE INVENTION

We have now surprisingly found that disruption of the gene encoding the virion host shut-off protein (vhs) in HSV vectors enables efficient dendritic cell activation to occur in HSV infected cells. Disruption of the UL43 gene is not also needed. It has previously been shown that HSV infected dendritic cells usually do not become activated either by infection itself, or by other stimuli (Salio et al 1999, Kruse et al 2000).

We have identified a previously unknown function of the vhs protein in preventing dendritic cell activation. Dendritic cell activation is defined as the up-regulation of certain cell surface markers as compared to the non-activated state. These markers include CD83 and CD86. Dendritic cell activation may be stimulated by treatment with lipopolysaccharide (LPS). LPS treatment of dendritic cells infected with HSV does not result in the up-regulation of CD83 or CD86. We have shown that LPS treatment of dendritic cells infected with a mutant HSV in which vhs is inactivated but which have a functional UL43 gene up-regulates both CD83 and CD86. Up-regulation of CD83 and CD86 is not observed following LPS treatment of dendritic cells infected with viruses comprising a functional vhs gene. Thus our results indicate that, for transduced dendritic cells to maximally stimulate an immune response following herpes virus infection, the gene encoding vhs should be disrupted but the gene encoding UL43 need not be.

Our results also demonstrate a role for vhs in the pathogenesis of wild type herpes simplex viruses. HSV infects dendritic cells at a high efficiency and it would seem likely that the reason it has evolved to do this as a part of its natural life-cycle is so that it can minimise a cell-mediated immune response which might otherwise prevent a latent HSV infection being efficiently established or result in clearance of the virus during repeated cycles of latency and reactivation. Dendritic cell activation is important in the stimulation of an effective cell-mediated immune response. Vhs is a virion protein and so, whilst HSV genes are generally not expressed at high levels in dendritic cells, the vhs protein would be delivered to the dendritic cell along with the incoming virus. Thus the novel function of vhs in preventing activation of dendritic cells infected with HSV is likely to be an important function of vhs in the HSV lifecycle following infection of a human with HSV.

Accordingly, the present invention provides the use of an attenuated herpes virus which:
(i) lacks a functional vhs gene, or a functional equivalent thereof; and
(ii) comprises a functional UL43 gene, or functional equivalent thereof;
in the manufacture of a medicament for use in a method of stimulating an immune response, which method comprises infecting dendritic cells with the said virus. Preferably said virus is a human herpes simplex virus. More preferably, said virus is HSV1 or HSV2. The dendritic cells may be infected *in vitro* or *in vivo.*

The virus may contain one or more additional mutation. The additional mutations preferably minimise the toxicity of the virus. Typically such mutations result in reduced or minimised immediate early (IE) gene expression. Prevention or reduction of IE gene expression prevents or reduces virus replication. Such mutations include, for example, inactivating mutations in the genes encoding ICP4, ICP27, ICPO and/or ICP22, preferably ICP27 and/or ICP4. An inactivating mutation in the vmw65-encoding gene removing its transactivating function may also be included (e.g. vmw65 mutations as in Ace *et al*., 1989 or Smiley *et al* 1997). Preferably the additional mutations may also minimise the immune response-inhibitory activity of the virus. Such mutations include inactivation of the gene encoding ICP47.

### BRIEF DESCRIPTION OF THE DRAWINGS

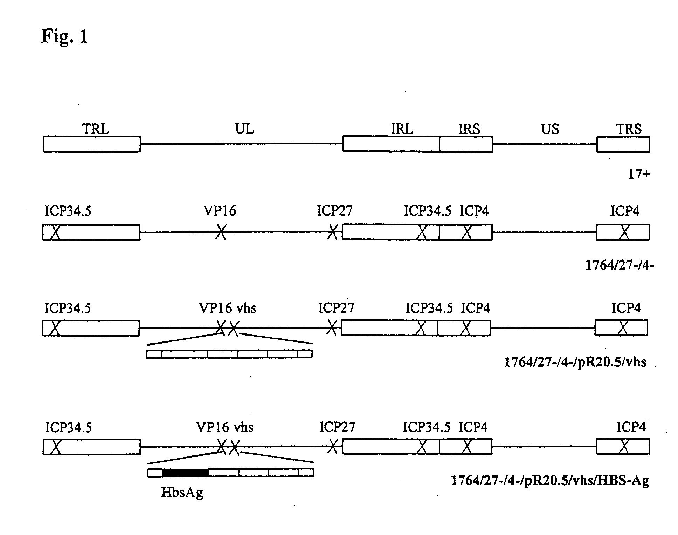

Figure 1 shows the viral strains 1764/27-/4-, 1764/27-/4-/pR20.5/vhs, 1764/27-/4-/pR20.5/vhs/HBS-Ag and wild type HSV strain 17+.

Figure 2 shows the results of FACS analysis to determine the levels of cell-surface expression of CD40, CD80, CD83 and CD86 on un-stimulated (figure 2A) and LPS stimulated (figure 2B) mock infected cells and cells infected with 17⁺, 1764/27-/4- and 1764/27-/4-/pR20.5/vhs viral strains.

Figure 3 shows the results of ELISA analysis to determine the effect of viral infection on IL-6 and TNFα secretion from uninfected dendritic cells and dendritic cells infected with 17⁺, 1764/27-/4- and 1764/27-/4-/pR20.5/vhs viral strains.

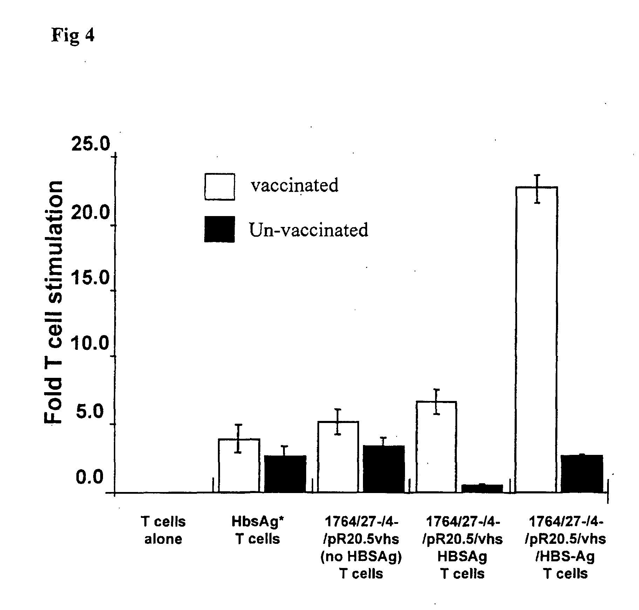

Figure 4shows the proliferative responses of T-cells prepared from hepatitis-B vaccinated and un-vaccinated individuals in response to HBS-Ag. Dendritic cells taken from each individual were either untreated, mixed with recombinant HBS-Ag protein (HBSAg*), infected with the control vector (1764/27-/4-/pR20.5/vhs HBSAg) or infected with the vector expressing HBS-Ag (1764/27-/4-/pR20.5/vhs/HBS-Ag) before mixing with the T cells.

### DETAILED DESCRIPTION OF THE INVENTION

### A. Viruses

A virus of the invention is capable of infecting dendritic cells without preventing the infected dendritic cells from being activated. Preferably dendritic cells infected with a virus of the invention at a multiplicity of infection (MOI) of 1 can be activated by treatment with LPS or other activation stimuli.

A virus of the invention does not prevent the activation of dendritic cells. To determine when a virus allows the activation of dendritic cells to occur, dendritic cells are infected with the virus at a MOI≥1 and infected dendritic cells are treated with LPS. The levels of cell surface markers such as CD83 and/or CD86 which are up-regulated on activation of dendritic cells may be monitored to determine dendritic cell activation, for example by FACS analysis. The level of these markers on the cell surface will be significantly higher in LPS treated dendritic cells than in cells which have not been treated with LPS if the virus with which the cells are infected allows dendritic cell activiation to occur. Some or all of these markers will also be higher on dendritic cells which have been infected with a virus that allows dendritic cell activation to occur compared to uninfected dendritic cells. If a herpes simplex virus which does not contain an inactivating mutation in vhs is used to infect dendritic cells, significantly less up-regulation of these markers is observed.

To permit activation of infected dendritic cells to occur, a virus of the invention will lack a functional gene encoding vhs (in HSV) or homologues or functional equivalents thereof in other viral species. In addition, a virus of the invention will have a functional UL43 gene. Additional mutations may be made to reduce further the immune-response-inhibitory effects of the virus for example by the inclusion of a mutation in the gene encoding ICP47.

An attenuated virus of the invention is preferably capable of infecting dendritic cells such that minimal toxicity results. Preferably cell survival post-infection will be at least 50% at one day post-infection, more preferably at least 60, 70, 80 or 90% at one day post-infection. To achieve reduced toxicity one or more mutations which reduce viral replications may be included in a virus of the invention. For example, a mutation in the gene encoding VMW65 which mutation minimises the trans-activating activity of the protein and/or a mutation in one or more regulatory immediate early gene such as ICP4, ICP27, ICP0 and ICP22 may be included. Thus a virus of the invention may typically lack functional vhs, ICP27, ICP4 genes and comprise a VMW65 gene which encodes a protein which does not exhibit transcriptional-activation activity, or may typically lack functional vhs, ICP47 and ICP4 genes.

For direct use *in vivo* some degree of replication competence may typically be beneficial in boosting the immune responses induced. Thus in these circumstances, a virus of the invention preferably lacks a functional vhs gene and may also lack one or more functional genes which are necessary for full pathogenicity of the virus but which are not necessary for viral replication. Such genes include those encoding ICP34.5, ICP6, thymidine kinase and glycoproteins such as gH. Preferably, however, the gene encoding thymidine kinase is functional as mutation of this gene would render the virus insensitive to anti-viral agents such as acyclovir.

Although the present invention has been exemplified using herpes simplex viruses, it will be understood that other viruses of the herpesviridae family may be modified to reduce the prevention of dendritic cell activiation of infected dendritic cells. In particular, such viruses may include varicella zoster virus, pseudo-rabies virus or bovine herpes viruses.

When the virus of the invention is a herpes simplex virus, the virus may be derived from, for example, HSV1 or HSV2 strains, or derivatives thereof, preferably HSV1. Derivatives include inter-type recombinants containing DNA from HSV1 and HSV2 strains. Such inter-type recombinants are described in the art, for example in Thompson *et al* (1988) and Meignier *et al* (1988). Derivatives preferably have at least 70% sequence homology to either the HSV1 or HSV2 genomes, more preferably at least 80%, even more preferably at least 90 or 95%, typically as measured by the methods described herein. More preferably, a derivative has at least 70% sequence identity to either the HSV1 or HSV2 genome, more preferably at least 80% identity, even more preferably at least 90%, 95% or 98% identity.

A derivative may have the sequence of a HSV1 or HSV2 genome modified by nucleotide substitutions, for example from 1, 2 or 3 to 10, 25, 50 or 100 substitutions. The HSV or HSV2 genome may alternatively or additionally be modified by one or more insertions and/or deletions and/or by an extension at either or both ends.

Derivatives which may be used to obtain the viruses of the present invention include strains that already have mutations in genes which it is desired to functionally inactivate in a virus of the invention, for example vhs inactivated strains (as in Jones *et al*. 1995), ICP47 inactivated strains (as in Goldsmith *et al*. 1998), strain d120 which has a deletion in ICP4 (DeLuca *et al*., 1985), strain d27-1 (Rice and Knipe, 1990) which has a deletion in ICP27) or strain d92 which has deletions in both ICP27 and ICP4 (Samaniego *et al*., 1995). Use of these strains will reduce the number of steps required to produce the mutant HSV strains of the present invention.

The terminology used in describing the various HSV genes is as found in Coffin and Latchman, 1996.

Where gene homologues of the HSV genes described above exist in other herpes virus species, then these homologues will be modified. By a "homologue" it is meant a gene which is functionally equivalent to a HSV gene a homologue typically exhibits sequence homology, either amino acid or nucleic acid sequence homology, to the corresponding HSV gene. Typically, a homologue of an HSV gene will be at least 15%, preferably at least 20%, more preferably at least 30%, 40% or 50% identical at the amino acid level to the corresponding HSV gene.

The geen encoding vhs is the UL41 gene in HSV1 and HSV2. In HSV1 strain 17+ (EMBL accession No. HE1CG) the UL41 gene is from nucleotide 91,170 to nucleotide 92,637. In HSV2 strain HG52 (EMBL accession No. z86099) the UL41 gene is from nucleotide 91,800 to nucleotide 93,275.

Methods of measuring nucleic acid and protein homology are well known in the art. For example the UWGCG Package provides the BESTFIT program which can be used to calculate homology (for example used on its default settings) (Devereux *et al.* (1984) *Nucleic Acids Research* **12**, p387-395). The PILEUP and BLAST algorithms can be used to calculate homology or line up sequences (typically on their default settings), for example as described in Altschul (1993) J. Mol. Evol. 36:290-300; Altschul *et al*. (1990) J. Mol. Biol. 215:403-10.

Software for performing BLAST analyses is publicly available through the National Centre for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al*., 1990). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) *Proc. Natl. Acad*. *Sci.* USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) *Proc. Natl*. *Acad. Sci.* USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Homologues of HSV genes can be identified in a number of ways, for example by probing genomic'or cDNA libraries made from other viruses with probes comprising all or part of the HSV gene under conditions of medium to high stringency (for example 0.03M sodium chloride and 0.03M sodium citrate at from about 50°C to about 60°C). Alternatively, species homologues may also be obtained using degenerate PCR which will use primers designed to target sequences within the variants and homologues encoding conserved amino acid sequences. The primers will contain one or more degenerate positions and will be used at stringency conditions lower than those used for cloning sequences with single sequence primers against known sequences (for example 0.03M sodium chloride and 0.03M sodium citrate at about 40°C).

A homologue in a herpes virus is a functional equivalent of an HSV protein if it shares one ore more functional characteristics with the HSV protein. For example, a vhs protein plays a role in reducing protein expression levels in an infected cell by reducing the stability of mRNA. Therefore, a functional equivalent of vhs protein preferably plays a role in shutting down host-cell gene expression by reducing the stability of mRNA. More preferably, a functional equivalent of vhs prevents dendritic cell activation in response to stimuli which activate un-infected dendritic cells.

For reasons of safety, the viruses of the invention are attenuated, typically so that they are incapable of causing disease Viral regions altered for the purposes of attenuation may be either eliminated (completely or partly), or made non-functional, or substituted by other sequences, in particular by a heterologous gene sequence. Attenuating mutations have been described for all viral groups used as viral vectors. For example, HSV may be rendered avirulent by mutations in ICP34.5 and/or essential genes such as ICP4,ICP27 and/or the vhs gene itself.

Particularly preferred attenuated viruses include viruses which, in addition to lacking a functional gene encoding vhs and optionally lacking a functional ICP47 gene, lack a functional ICP34.5 gene and a functional ICP27 gene and optionally lacks a functional ICP4 gene and/or a VMW65 gene which encodes a protein which has transcriptional-activation activity, and viruseswhich have a functional ICP27 gene but lack a functional ICP4 gene and a functional ICP34.5 gene and optionally lacks a VMW65 gene which encodes a protein which has transcriptional-activation activity. Such viruses are described in WO98/04726 and WO99/60145, the disclosures of which are herein incorporated by reference.

When a herpes simplex virus of the invention lacks a particular functional essential gene, for example a gene encoding ICP4 or ICP27, the virus is propagated using a cell line expressing that essential gene. For example, when the virus lacks a functional ICP27 gene, the virus may be propagated using V27 cells (Rice and Knipe, 1990), 2-2 cells (Smith *et al*., 1992) or B130/2 cells (WO98/30707), preferably B130/2 cells. When the virus lacks a functional ICP4 gene the virus may be propagated using a cell line expressing ICP4, for example E5 cells (DeLuca *et al*., 1985). When the virus lacks a functional ICP4 gene and a functional ICP27 gene the virus is propagated using a cell line expressing both ICP4 and ICP27 (such as E26 cells; Samaniego *et al*., 1995), and when the virus additionally lacks a functional vmw65 gene the virus may be propagated using a cell line also containing a non-HSV homologue of vmw65 (e.g. equine herpes virus gene 12 or BTIF from bovine herpes virus).

### B. Methods of mutation

The various viral genes referred to may be rendered functionally inactive by several techniques well known in the art. For example, they may be rendered functionally inactive by deletion(s), substitution(s) or insertion(s), preferably by deletion. A deletion may remove portions of a gene or the entire gene. For example, deletion of only one nucleotide may be made, resulting in a frame shift. However, preferably larger deletions are made, for example from 2, 3 or 5 to 10, 20, 30, 50, 100 or 200 nucleotide substitutions. Preferably at least 25%, more preferably at least 50% of the total coding and non-coding sequence (or alternatively, in absolute terms, at least 10 nucleotides, more preferably at least 100 nucleotides, most preferably, at least 1000 nucleotides) is deleted or substituted. It is particularly preferred to remove the entire gene and some of the flanking sequences. Inserted sequences may include the heterologous genes described below. Mutations may comprise both deletion(s) and insertion(s). For example, an insertion may be made into the site of a deletion. Thus insertion of a heterologous gene into a viral gene may replace part or all of the viral gene. In particular, it is preferred to insert the heterologous gene into vhs, ICP47, ICP27 or ICP4. In the case of the VMW65 gene, the entire gene is not deleted since it encodes an essential structural protein, but an inactivating mutation is typically made which abolishes the ability of VMW65 to activate transcriptionally IE genes (e.g. as in Ace *et al*., 1989 or Smiley *et al*., 1997).

Mutations may be made in the herpes viruses by homologous recombination methods well known to those skilled in the art. For example, HSV genomic DNA is transfected together with a vector, preferably a plasmid vector, comprising the mutated sequence flanked by homologous HSV sequences. The mutated sequence may comprise deletions, insertions or substitutions, all of which may be constructed by routine techniques. Insertions may include selectable marker genes, for example lacZ or GFP, for screening recombinant viruses by, for example, β-galactosidase activity or fluorescence.

### C. Heterologous genes and promoters

The viruses of the invention may be modified to carry a heterologous gene/genes. The term "heterologous gene" encompasses any gene. Although a heterologous gene is typically a gene not present in the genome of a herpes virus, a herpes gene may be used provided that the coding sequence is not operably linked to the viral control sequences with which it is naturally associated. The heterologous gene may be any allelic variant of a wild-type gene, or it may be a mutant gene. The term "gene" is intended to cover nucleic acid sequences which are capable of being at least transcribed to produce an RNA molecule, which RNA molecule is preferably capable of being translated to produce a polypeptide or to down-regulate gene expression levels by an anti-sense effect A virus of the invention may optionally include some or all of 5' and/or 3' transcribed but untranslated flanking sequences naturally, or otherwise, associated with the translated coding sequence of a heterologous gene. It may optionally further include the associated transcriptional control sequences normally associated with the transcribed sequences, for example transcriptional stop signals, polyadenylation sites and downstream enhancer elements.

The heterologous gene/genes may be inserted into the viral genome by homologous recombination of HSV strains with, for example, plasmid vectors carrying the heterologous gene/genes flanked by HSV sequences. The heterologous gene/genes may be introduced into a suitable plasmid vector comprising herpes viral sequences using cloning techniques well-known in the art. The heterologous gene/genes may be inserted into the viral genome at any location provided that the virus can still be propagated. It is preferred that the heterologous gene/genes is inserted into a gene resulting in attenuation of the virus. Heterologous genes may be inserted at multiple sites within the virus genome.

The transcribed sequence of the heterologous gene/genes is preferably operably linked to a control sequence permitting expression of the heterologous gene/genes in dendritic cells, preferably mammalian dendritic cells, more preferably human dendritic cells. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence.

The control sequence comprises a promoter allowing expression of the heterologous gene/genes and a signal for termination of transcription. The promoter is selected from promoters which are functional in mammalian, preferably human dendritic cells. The promoter/promoters may be derived from promoter sequences of eukaryotic genes. For example, promoters may be derived from the genome of a cell in which expression of the heterologous gene is to occur, preferably a mammalian dendritic cell or more preferably a human dendritic cell. With respect to eukaryotic promoters, they may be promoters that function in a ubiquitous manner (such as promoters of β-actin, tubulin) or, alternatively, a dendritic cell-specific manner. Viral promoters may also be used, for example the Moloney murine leukaemia virus long terminal repeat (MMLV LTR) promoter or other retroviral promoters, the human or mouse cytomegalovirus (CMV) IE promoters.

Expression cassettes and other suitable constructs comprising the heterologous gene/genes and control sequences can be made using routine cloning techniques known to persons skilled in the art (see, for example, Sambrook *et al*., 1989, Molecular Cloning - a laboratory manual; Cold Spring Harbor Press).

In addition, any of these promoters may be modified by the addition of further regulatory sequences, for example enhancer sequences (including elements of the HSV LAT region). Chimeric promoters may also be used comprising sequence elements from two or more different promoters described above, for example an MMLV LTR/LAT fusion promoter (Lokensgard *et al*., 1994) or promoters comprising elements of the LAT region (WO98/30707).

Heterologous genes will typically encode polypeptides of therapeutic use. For example, to promote an immune response specifically against a particular tumour, it will be desirable to transfect dendritic cells with a virus of the invention directing expression of a tumour antigen/antigens. A tumour antigen may be specific to a tumour cell, i.e. present in tumour cells but not in non-tumour cells, or it may be present at higher levels in that tumour cell than in a non tumour cell of that type, for example due to up regulation of expression of the antigen. This will be useful in cancer therapy since an infected dendritic cell of the invention can be used to stimulate the host immune system to react to the tumour-specific or tumour-prevalent antigen/antigens resulting in tumour reduction/regression. In particular, it is preferred that the tumour antigen/antigens is expressed on the surface of the tumour cell, for example a cell surface receptor or cell adhesion protein. Examples of tumour antigens include the MUC-1 gene product (Gendler *et al*., 1990) which is over expressed in a number of tumours including ovarian cancers, human papillomavirus proteins E6 and E7 which are associated with cervical cancer. MART-I, MAGE-I, gp100 and tyrosinase in melanoma, PSA in prostate cancer, CEA in a number of different types of tumour and Her2neu in various cancers including breast cancer.

Heterologous genes may also encode a polypeptide which is capable of modifying an immune response, for example cytokines (such as α-, β- or γ-interferon, interleukins including IL-1, IL-2, tumour necrosis factor, or insulin-like growth factors I or II) or other immunomodulatory proteins including chemokines such as RANTES and co-stimulatory molecules such as CD80, CD86, CD40 and CD40 ligand.

The heterologous gene may also encode a polypeptide/polypeptides of pathogenic origin so that, for example, a dendritic cell infected with a virus of the invention can be used to stimulate the host immune system to produce an immune response to a pathogen, either prior to infection or after infection of the host by the pathogen. Viruses for use in vaccines may typically comprise heterologous genes that encode antigenic polypeptide(s). Preferably such polypeptides of pathogenic origin are derived from pathogenic organisms, for example parasites, bacteria or viruses. Examples of such antigenic polypeptides include hepatitis C virus antigens, hepatitis B surface or core antigens, papillomavirus antigens, HTV antigens and malaria antigens. Viruses comprising heterologous genes from pathogenic organisms may be used for either or both therapeutic and prophylactic treatment.

Therapeutic applications may well require the administration of multiple genes. The expression of multiple genes may be advantageous for the treatment of a variety of conditions. Herpes viruses are uniquely appropriate as they do not have the limited packaging capabilities of other viral vector systems. Thus multiple heterologous genes can be accommodated within its genome. For example, from 2 to 6 genes may be inserted into the genome.

There are, for example, at least two ways in which this could be achieved. For example, more than one heterologous gene and associated control sequences could be introduced into a particular HSV strain either at a single site or at multiple sites in the virus genome. It would also be possible to use pairs of promoters (the same or different promoters) facing in opposite orientations away from each other, these promoters each driving the expression of a heterologous gene (the same or different heterologous gene) as described above.

### D. Dendritic cells

Dendritic cells can be isolated/prepared by a number of means, for example they can either be purified directly from peripheral blood, or generated from CD34+ precursor cells for example after mobilisation into peripheral blood by treatment with G-CSF, or directly from bone marrow. From peripheral blood adherent precursors can be treated with a GM-CSF/IL-4 mixture (Inaba *et al*., 1992), or from bone marrow non-adherent CD34+ cells can be treated with GM-CSF and TNF-α (Caux *et al*., 1992). DCs can be routinely prepared from the peripheral blood of human volunteers, similarly to the method of Sallusto and Lanzavecchia, 1994, using purified peripheral blood mononeucleocytes (PBMCs) and treating 2 hour adherent cells with GM-CSF and IL-4. These are then depleted of CD19+ B cells and CD3+, CD2+ T cells using magnetic beads (see Coffin *et al*., 1998). Other methods may also be used for the preparation of dendritic cells.

### E. Therapeutic uses

Viruses of the invention, and dendritic cells infected with viruses of the invention may be used in methods of therapy. In particular, viruses of the invention, and dendritic cells infected with viruses of the invention, which express tumour antigens may be used in methods of treating cancer. Specifically, the, viruses of the invention, and dendritic cells infected with viruses of the invention may be used to inhibit the growth of various tumours in mammals, including humans, such as, for instance, ovarian, cervical and endometrial tumours and carcinomas, for example mammary carcinoma, lung carcinoma, bladder carcinoma and colon carcinoma. Other neoplasms whose growth may be inhibited include sarcomas, for example soft tissue and bone sarcomas, and hematological malignancies such as leukemias. Particular examples of cancers which may be treated using viruses of the invention and/or dendritic cells infected with viruses of the invention which express tumour antigens include melanomas, leukemias, cervical cancers and ovarian cancers. A virus for use in treating cancer typically comprises a heterologous gene encoding a tumour antigen. Administration of such a virus, or dendritic cells infected with such a virus, will typically result in the generation of an immune response to the tumour antigen.

Viruses of the invention, and dendritic cells infected with viruses of the invention, may be used in methods of treating or preventing pathogenic infections, for example parasitic, bacterial or viral infections. A virus for use in treating a pathogenic infection typically comprises a heterologous gene encoding an antigen from the pathogenic organism. Administration of such a virus, or dendritic cells infected with such a virus, will typically result in the generation of an immune response to antigen from the pathogenic organism. Such viral infections include herpes virus infections. Thus, a virus of the invention may be used to induce immune responses to the virus itself, for example in the treatment or vaccination of HSV1 or HSV2 infection. Where a virus is intended for use in the treatment of HSV1 or HSV2, the virus may optionally contain a heterologous gene, which heterologous gene encodes an HSV antigen (which is not under the control of its natural promoter) or an immunomodulatory molecule. The viruses/dendritic cells may be administered prior to infection to stimulate a protective immune response in the host, or after infection to stimulate the host immune system to combat the infection.

### F. Administration

The herpes viruses of the present invention may thus be used to deliver therapeutic genes to a human or animal in need of treatment. Delivery of therapeutic genes using the herpes viruses of the invention may be used to treat for example, malignancies and/or pathogenic infections.

The viruses of the invention may be used in a patient, preferably a human patient, in need of treatment. A patient in need of treatment is an individual suffering from cancer, or a patient with a pathogenic infection. The aim of therapeutic treatment is to improve the condition of a patient. Typically therapeutic treatment using a virus of the invention allieviates the symptoms of the cancer. A method of treatment of cancer according to the invention comprises administering a therapeutically effective amount of a virus having a functional UL43 gene and lacking a functional vhs gene to a patient suffering from cancer such that the virus is present in dendritic cells in the patient. Administration of virus of the invention to an individual suffering from a tumour will typically kill the cells of the tumour thus decreasing the size of the tumour and/or preventing spread of malignant cells from the tumour.

Typically therapeutic treatment of a pathogenic infection using a virus of the invention alleviates the symptoms of the infection and preferably kills the pathogenic organism. A method of treatment of a pathogenic infection according to the invention comprises administering a therapeutically effective amount of a virus lacking a functional vhs gene to a patient with a pathogenic infection. Preferably the virus enters dendritic cells in the patient or dendritic cells which have been infected with the virus *ex vivo* are administered to the patient. Prophylactic treatment using a virus of the invention typically leads to the production of antibodies against a tumour antigen or against an antigen from a pathogenic organism in a patient at risk of cancer or a pathological infection. Typically a patient at risk of cancer may be genetically disposed thereto or may have been exposed to or be at risk of exposure to a carcinogen. Typically a patient at risk of a pathogenic infection may be likely to be exposed to a pathogenic organism.

One method for carrying out therapy involves inserting the therapeutic gene/genes into the genome of the herpes virus of the invention, as described above, and then combining the resultant recombinant virus with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, intraperitoneal, subcutaneous or transdermal administration. Subcutaneous or intraperitoneal administration is preferred. Trans- or intradermal administration may be particularly preferred.

Infection of dendritic cells with the virus of the invention may be carried out *in vivo* by administration of a composition comprising the virus to a patient. The pharmaceutical composition is administered in such a way that the virus containing the therapeutic gene/genes, can infect dendritic cells. The amount of virus administered is in the range of from 10⁴ to 10¹⁰ pfu, preferably from 10⁵ to 10⁸ or from10⁵ to 10⁹ pfu, more preferably about 10⁶ to 10⁸ pfu. When injected intra-dermally or trans-dermally administered, for example using a needle-free device, typically from 10 µl to 1 ml, preferably from 100 µl to 1 ml of virus in a pharmaceutically acceptable suitable carrier or diluent or in a particulate composition is administered

Another method involves isolating/preparing dendritic cells from peripheral blood or bone marrow and infecting the cells with the virus of the invention *in vitro*. Transduced dendritic cells are then typically administered to the patient by intramuscular, intraperitoneal, subcutaneous or intravenous injection, or by direct injection into the lymph nodes of the patient, preferably by subcutaneous, intraperitoneal or direct injection into the lymph nodes. Typically from 10⁵ to 10⁹ transduced dendritic cells, preferably from 10⁶ to 10⁸ cells, more preferably about 10⁷ cells are administered to the patient.

The routes of administration and dosages described are intended only as a guide since a skilled practitioner will be able to determine readily the optimum route of administration and dosage for any particular patient. The dosage may be determined according to various parameters, especially according to, for example, the age, weight and condition of the patient.

The following Examples illustrate the invention.

### EXAMPLES

### Materials and Methods

### Construction and Growth of Viral Strains

All virus strains are derived from HSV1 strain 17+, the nucleotide sequence of which is deposited in GenBank (Accession No. HE1CG). Viral strains were produced and propagated using BHK C-21 cells (ECACC No. 8501143) or BHK cells stably transfected with the genes encoding HSV1 ICP27, ICP4 and equine herpes virus gene 12 (Thomas *et al*. 1999).

For viruses with mutations in VMW65, 3mM hexamethylene-bisacetamide (HMBA) was included in the media used for virus growth (McFarlane *et al*., 1992). The following viral strains were used.
*(i) 17+ (wild type HSVI)*
*(ii) 17+*/*pR20.5*/*UL43*
   A cassette from plasmid pR20.5 (Thomas *et al*. 1999b) consisting of an RSV/lacZ/pA sequence and a CMV/GFP/pA sequence in opposite back-to-back orientations and separated by an HSV LAT region sequence (nts 118,866-120,219) was inserted into the UL43 locus by homologous recombination with purified genomic HSV1 strain 17+ DNA by standard methods. The pR20.5 cassette was first inserted into a plasmid containing UL43 flanking regions (Coffin *et al*, 1996) at the unique Nsil site, giving plasmid pR20.5/43. The 20.5 cassette can be excised from its pGEM5 (Promega) plasmid backbone with Srfl as an oligonucleotide encoding Srfl was inserted on either side of the cassette. The RSV promoter was excised from pRc/RSV (Invitrogen), lacZ/pA from pCH110 (Pharmacia), CMV/pA from pcDNA3 (Invitrogen) and GFP from pEGFP-N1 (Clontech) for the construction of plasmid pR20.5.
*(iii) 1764*/*27-*/*4-*
   Virus strain 1764/27-/4- was constructed by recombination of virus strain 1764/27-/4-/pR20.5 DNA with empty ICP4 flanking regions and the selection of virus plaques which do not express GFP or lacZ. Virus strain 1764/27-/4-/pR20.5 is described in Thomas *et al*. 1999b and contains the pR20.5 cassette inserted into the ICP4 geneso as to replace the gene encoding ICP4 of a virus also deleted for ICP27 and ICP34.5 and with an inactivating mutation in the gene encoding VMW65.
(iv) *1764*/*27-*/*4-*/*pR20.5*/*vhs*
   Virus strain 1764/27-/4-/pR20.5/vhs was constructed by insertion of the pR20.5 cassette into *vhs* flanking regions at the unique NruI site in the vhs encoding gene of HSV1 strain 17+ and the resulting plasmid (pR20.5/vhs) was recombined into HSV strain 1764/27-/4- DNA. Virus strain 1764/27-/4-/pR20.5/vhs is therefore deleted for the genes encoding ICP4, ICP27 and ICP34.5, and has inactivating mutations in the genes encoding vmw65 and vhs.
(v) *1764*/*27-*/*4-*/*pR191acZ*
   Virus strain 1764/27-/4-/pR191acZ was constructed as for virus (iv) above except the pR19lacZ cassette (Wagstaff *et al.* 1998) was recombined into the latency associated transcript (LAT) region of virus strain 1764/27-/4- rather than the pR20.5 cassette into vhs.
*(vi) 1764*/*27-*/*4-*/*pR20.5*/*vhs*/*HBS-Ag*
   The lacZ gene in the pR20.5/vhs plasmid was replaced by the gene encoding hepatitis surface antigen (HBS-Ag) by digestion of pHBV130 (Gough and Murray, 1982) with XhoI and NsiI and insertion of the fragment released into pSP72 (Promega) between the SalI and Smal sites. pR20.5/vhs was digested with Xbal and EcoRI to release the lacZ gene which was replaced by the HBS-Ag gene excised from pSP72 with HindIII and EcoRI. The resulting plasmid was recombined into 1764/27-/4-/pR20.5/vhs viral DNA and non-lacZ expressing plaques selected and purified. Genome structure was then confirmed by Southern blot.

### Dendritic Cell preparation

DC were prepared from peripheral blood as previously described (Coffin et al 1998). Briefly, peripheral blood mononuclear cells (PBMCs) were prepared from 60ml of healthy/hepatitis B vaccinated donor blood using lymphoprep (Nycomed). After removal of red cells, non-adherent cells (mainly T cells and B cells) were removed, washed in HBSS and centrifuged at 1400 rpm, 5 minutes, RT. The cell pellet was resuspended in a 2 ml 90% FCS:10% dimethylsulphoxide (DMSO) mix, aliquoted and stored at -80 C for subsequent T cell isolation. Adherent cells were cultured in RPMI medium supplemented with GM-CSF (0.1 µg/ml) and IL-4 (0.05 µg/ml) and incubated for 7 days, at 37 C, 5% CO₂. After further lymphoprep purification cells were then magnetically depleted using anti-CD19, anti-CD2 (Harlan) and anti-CD3 (Harlan) antibodies and DC were resuspended in complete RPMI medium for immediate use.

### Isolation of CD4+ T-cells

T and B cell frozen as above were defrosted quickly, washed in HBSS and centrifuged at 1400 rpm, for 5 minutes. Cells were resuspended in 2 ml complete RPMI medium, counted and incubated with anti-CD19 (BU12 - 200 µl neat, Immunology dept, UCL), anti-CD14 (HB246 - 200 µl neat, Immunology dept, UCL) and anti-HLA-DR (L243 - 100 µl neat, Immunology dept, UCL) mAb and left on ice for 30 minutes. The cells were washed in HBSS, resuspended in 2 ml complete RPMI medium, mixed with sheep anti-mouse antibodies bound to magnetic beads (Dynabeads, Dynal) at a ratio of 10 µl beads/10⁶ contaminating cells and incubated on a rotor mixer at 4 C, for 45 minutes. CD4+ T cells were then depleted by removing the supernatant after placing the cell suspension/magnetic bead mix in contact with a magnet, for 10 minutes, on ice. CD4+ T cells were counted, resuspended in complete RPMI medium at the appropriate concentration, left on ice or cultured o/n at 37 C, 5% CO₂ for subsequent use.

### Infection of DC

DC were pelleted at 1400 rpm for 5 minutes at room temperature. DC were then infected at MOI of 1 by resuspension in RPMI medium containing virus for 1 hour at 37 C, 5% CO₂. 1 ml of RPMI supplemented with GM-CSF (0.1 µg/ml) and IL-4 (0.05 µg/ml) was then added and. DC incubated at 37 C, 5% CO₂. For LPS stimulation, RPMI additionally containing 100ng/ml LPS was used.

### Cytokine analysis

IL-6, and TNF-α were measured in DC culture supernatants using commercially available ELISA kits (R&D Systems). Prior to ELISA, supernatants were collected 42 hours post-infection of DC with the indicated viruses and stored at -20 C before use.

### T cell proliferation assays

DC and CD4+ T cells were isolated and treated as above from hepatitis B vaccinated and un-vaccinated human individuals. DC were used at dilutions from 1x10⁵ DC/ml to 1x10⁴ DC/ml and CD4+ T cells at 1x10⁶ cells/ml. Experiments at each of DC concentration were performed in triplicate. 100 µl of DC and 100 µl of CD4+ T cells were added to each assay well. Where indicated recombinant hepatitis B surface antigen (Austral) was added to wells at a final concentration of 1 µg/well. HSV-1-infected and uninfected DC were cultured with CD4+ T cells for 6 days at 37 C, 5% CO₂. 1 µCu/well [³H] thymidine (Amersham) was then added and 18 hours later cells harvested and [³H] thymidine incorporation counted.

### Example 1 Preliminary data showing that HSV strains not containing a functional vhs gene give enhanced activation of dendritic cells following virus infection.

Here in each case 1x10⁵ dendritic cells were infected with each of the viruses by gentle pelleting, resuspension in about 100 µl virus suspension in DMEM, incubation at 37°C for 1hr, and transfer into 24 well plates with 2ml of RPMI/10%FCS + 100 ng/ml GM-CSF, 50 ng/ml IL-4. These plates were then incubated at 37°C/5% CO₂ overnight. Dendritic cells were also treated with lipopolysaccharide (LPS) a known dendritic cell activator, and untreated as a controls.

Supernatants from these infections and from the control were then used in ELISA tests to detect levels of secreted cytokines. Fluorescence activated cell sorting (FACS) was also used to detect levels of expression of CD86 on the surface of infected and control dendritic cells. In dendritic cell cultures there are two populations of cells with respect to levels of CD86 expression. These are observed as two peaks by FACS analysis reflecting a first peak of cells with a relatively lower level of CD86 expression and a second peak of cells with a relatively higher level of CD86 expression. On activation by e.g. LPS more of the cells express higher levels of CD86 and there are thus more cells are found in the second peak.

### Results

**Table 1:**

| Cytokine concentration in culture supernatants 24hr after infection with the indicated viruses or in control supernatants at an MOI of 1. Measured by ELISA. | | |
|---|---|---|
| Treatment | Cytokine Concentration (ng/well) | |
| | IL-6 | TNFa |
| 17+/pR20.5/UL43 | 6 | 1.1 |
| 1764/27-/4-/pR19lacZ | 4 | 0.8 |
| 1764/27-/4-/pR20.5/vhs | 46 | 7.1 |
| No infection | 4 | 0.9 |
| | | |

**Table 2:**

| Expression of CD86 on control cells and cells infected with the indicated viruses. | | | |
|---|---|---|---|
| Treatment | % cells peak 1 | % of cells peak 2 | Mean fluorescence intensity peak 2 |
| 17+/pR20.5/UL43 | 43.35 | 46.85 | 5x10² |
| 17+/pR20.5/UL43 + LPS | 56.93 | 29.06 | |
| 1764/27-/4-/pR191acZ | 24.1 | 68.5 | 5x10² |
| 1764/27-/4-/pR191acZ + LPS | 52.71 | 35.32 | 7x10² |
| 1764/27-/4-/pR20.5/vhs | 27.81 | 64.61 | 1x10³ |
| 1764/27-/4-/pR20.5/vhs+ LPS | 39.52 | 52.40 | 9x10² |
| No infection | 48.95 | 31.45 | 1x10³ |
| No infection + LPS | 30.33 | 60.81 | 9x10² |

### Conclusions

The results show that untreated dendritic cells secrete minimal levels of the cytokines tested and have the expected "resting" levels of CD86 on their surface. Following LPS treatment cytokine levels are significantly stimulated and the level of surface expression of CD86 increases significantly. In the experiments above the mean fluorescence intensity in LPS treated cells is approximately 1x10³ by FACS analysis with an anti-CD86 antibody. These results are indicative that dendritic cells are in an activated state.

Following infection of dendritic cells with the indicated viruses it can clearly be seen that for activation of dendritic cells to occur by these assays the virus must contain an inactivating mutation in the gene encoding vhs. Viruses of varying levels of disablement have been used, and only the virus containing the vhs mutation gives significant activation of the dendritic cells by these assays. It can also be seen that if mutation to vhs is not included when cells are treated with LPS as well as infected with the indicated viruses, CD86 levels are not increased in as many cells as occurs by treatment with LPS alone. Also, unless vhs mutation is included, the mean fluorescence intensity of CD86 expressing cells as measured by FACS following virus infection is reduced from that seen if cells are treated with LPS. For maximum immune stimulation by dendritic cells it can thus be concluded that inactivating mutation(s) in the gene encoding vhs should be included.

### Example 2 HSV strains not containing a functional vhs protein do not block dendritic cell activation

Fluorescence activated cell sorting (FACS) was used to detect levels of expression of CD86, CD80, CD83 and CD40 on the surface of infected and control dendritic cells. Supernatants from the infections was used to assess levels of cytokines by ELISA.

### Results

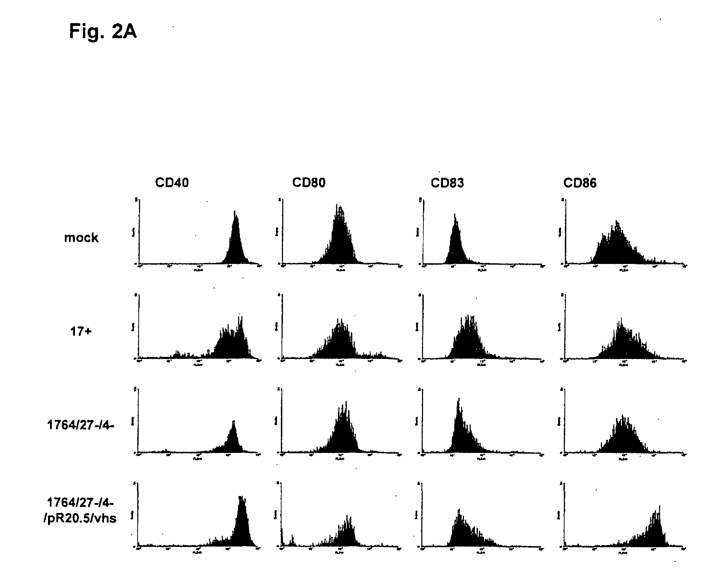

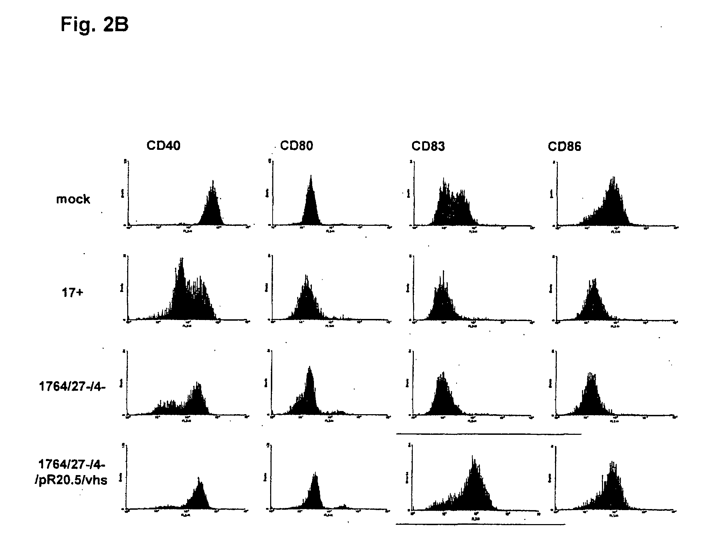

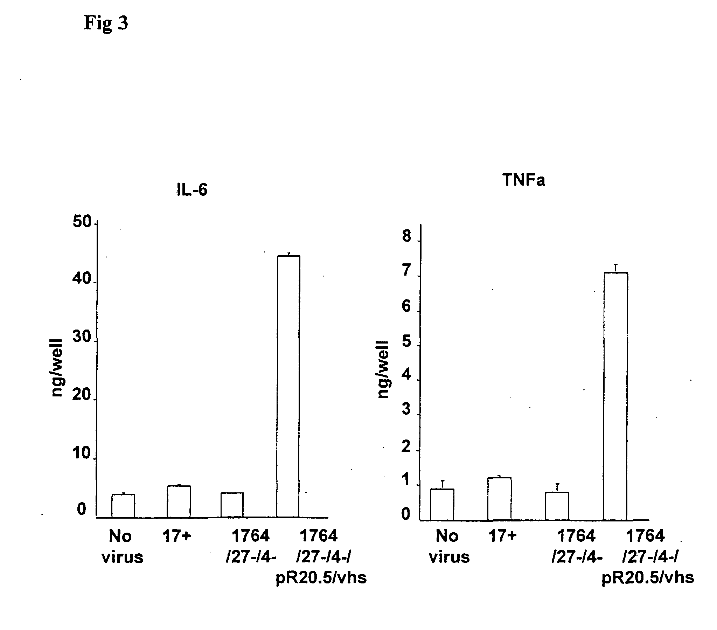

The ELISA results (figure 3) show that while DC can be infected with HSV at high efficiency, cytokines indicative of DC activation are not produced with either a wild type (strain 17+) or a disabled (strain 1764/27-/4-) virus. However if vhs is inactivated from strain 1764/27-/4- , giving strain 1764/27-/4-/pR20.5/vhs, cytokines indicative of DC activation are then produced.FACS analysis (figure 2) on non-LPS stimulated DC shows that infection with essentially wild type HSV (strain 17+) or a replication incompetent HSV vector (strain 1764/27-/4-) prevents the increased expression of CD86. As discussed above, increased CD86 expression would be expected if DC had become activated by the infection process. CD40 levels are also altered/reduced in HSV infected cells. However, if vhs is inactivated (strain 1764/27-/4-/pR20.5/vhs), CD86 levels are increased indicating activation, and CD40 levels are unaffected. CD80 and CD83 are not greatly affected in unstimulated DC infected with HSV. CD83 (B7.1) and CD86 (B7.2) are two key T-cell co-stimulatory molecules, CD40 is a key T-cell activator, and CD83 is a DC marker up-regulated during DC maturation and activation.

When DC are LPS stimulated at the time of infection effects on CD40 levels are more marked with both wild type (strain 17+) or disabled (strain 1764/27-/4-) virus. If vhs is inactivated, however, these effects on CD40 are prevented. LPS stimulated DC usually significantly up-regulate CD83 and CD86 expression, but this is blocked by HSV (strains 17+ and 1764/27-/4-) unless vhs is inactivated (strain 1764/27-/4-/pR20.5/vhs). When vhs is inactivated, both CD83 and CD86 levels are increased to a similar or greater extent as in LPS stimulated but uninfected cells.

### Conclusion

As in the preliminary experiments (Example 1), it can clearly be seen that for dendritic cells to become activated as measured by surface marker expression levels in response to HSV infection or HSV infection and LPS stimulation it is clear that the gene encoding vhs must be inactivated. Viruses encoding functional vhs do not allow dendritic cells to become significantly activated as measured by the expression levels of the surface markers tested.

### Example 3 DC transduced with a vhs inactivated HSV vector direct antigen specific T cell responses in vitro.

The results above suggested that HSV vectors in which vhs is inactivated might be used as effective vectors for DC as the inactivating effects of HSV in DC have been prevented. Indeed DC infected with such HSV mutants appear to be specifically activated in response to infection as measured by CD86 up-regulation and the secretion of certain cytokines. To test whether vhs-inactivated HSV mutants might be used to direct antigen specific immune responses following the delivery of antigen encoding genes to DC, experiments were performed using DC and T-cells prepared from hepatitis B vaccinated and un-vaccinated individuals. Here a virus was first constructed in which a hepatitis B surface antigen (HBS-Ag) expression cassette was inserted into the vhs encoding gene of the IE gene deficient virus. T-cell proliferation assays were then performed in which DC from vaccinated or un-vaccinated individuals were either untreated, 'loaded' with antigen by mixing with recombinant HBS-Ag protein, infected with the control marker gene containing vector (1764/27-/4-/pR20.5/vhs at MOI=1), infected with the control vector (1764/27-/4-/pR20.5/vhs at MOI=1) and also mixed with recombinant HBS-Ag, or infected with the vector expressing HBS-Ag (1764/27-/4-/pR20.5/vhs/HBS-Ag at MOI=1). DC were then mixed with T-cells derived from the same vaccinated or unvaccinated individuals respectively, and effects on T-cell proliferation observed in standard T-cell proliferation assays.

These experiments showed (Fig. ) that while HBS-Ag recombinant protein and the control HSV vector could induce a small T-cell proliferative response in vaccinated individuals, the HSV response probably indicating proliferation of T-cells specific to HSV structural proteins, and the control vector mixed with recombinant HBS-Ag could illicit a slightly greater response, the HBS-Ag expressing vector gave a significantly greater response than any of these. Thus following delivery of HBS-Ag directly into DC using an HSV vector a significant and specific T-cell proliferative response was induced which did not occur following mixing with recombinant antigen alone. HSV vectors with vhs inactivated thus allow the delivery of antigen coding genes to DC such that DC retain the ability to stimulate antigen specific T-cell proliferative responses.

### REFERENCES

- Gendler, S.J. *et al*.,(1990), J. Biol. Chem. 265:15286-15293.
- Aicher, A. *et al*., (1997), Exp. Hematol. 25:39-44.
- Samaniego, L.A. *et al.,* (1995), J. Virol. 69:5705-5715.
- *Zitvogel, L. et al*., (1996), J. Exp. Med 183:87-97.
- Celluzzi, C.M. *et al*., (1996), J. Exp. Med. 183:283-287.
- Reeves, M.E. *et al*., (1996), Cancer Research 56:5672-5677.
- Arthur, J.F. *et al*., (1997), Cancer Gene Therapy 4:17-25.
- Coffin, R.S. *et al*., (1998), Gene Therapy 5:718-722.
- Coffin, R.S. and Latchman, D.S. (1996), Genetic Manipulation of the Nervous System (D.S. Latchman Ed.) pp 99-114: Academic Press, London.
- Inaba, K. *et al*., (1992) J. Exp. Med. 175:1157-1167.
- Caux, C. *et al*., (1992), Nature 360:258-261.
- Sallusto, F. and Lanzavecchia, A. (1994), J. Exp. Med. 179:1109-1118.
- Coffin, R.S. *et al*., (1996), Gene Therapy 3:886-891.
- Ace, C.I. *et al*., (1989), J. Virol. 63:2260-2269.
- Smith, I.L. *et al*., (1992), Virology 186:74-86.
- Rice, S.A. and Knipe, D.M. (1990), J. Virol. 64:1704-1715.
- DeLuca, N.A. *et al*., (1985), J. Virol. 56: 558-570.
- MacFarlane, M. *et al*., (1992), J. Gen. Virol. 73:285-292.
- Lokensgard, J.R. *et al*., (1994), J. Virol. 68:7148-7158.
- Smiley, J.R. and Duncan, J. (1997), J. Virol. 71:6191-6193.
- Thomas, S.K. *et al*., 1999 J.Viol 73:7399-7409.
- Thomas, S.K. *et al*. 1999b J.Viol 73:6618-6625
- Wagstaff, M.J.D. *et al*. 1998 Gene Therapy 5:1566-1570
- Dilloo, D *et al*. 1997 Blood 89:119-127.
- Jones, F.E. *et al*. 1995 J. Virol 69: 4863-4871
- Goldsmith, K *et al*. 1998 J. Exp Med. 87: 341-348
- Salio et al. 1999 Eur. J. Immunol. 29: 3245-3253
- Kruse et al 2000 J. Virol. 74, 7127-7136
- Gough N. and K. Murray, J. Mol. Biol. 1982. 162, 43-67

## Claims

1. Use of an attenuated herpes virus which
(i) lacks a functional *vhs* gene, or a functional equivalent thereof;
(ii) comprises a functional UL43 gene, or a functional equivalent thereof;
in the manufacture of a medicament for use in a method of stimulating an immune response, which method comprises infecting dendritic cells with the said virus.

2. Use according to claim 1 wherein said virus is a herpes simplex virus 1 or 2.

3. Use according to claim 1 or 2 wherein said virus lacks a functional gene encoding ICP47.

4. Use according to any one of preceding claims wherein said virus has a VMW65 gene, or a functional equivalent thereof, which encodes a protein which lacks transcriptional-activation activity.

5. Use according to any one ofthe preceding claims wherein said virus lacks at least one functional immediate early gene.

6. Use according to claim 5 wherein said immediately early gene is selected from genes encoding ICPO, ICP4, ICP22, ICP27 or functional equivalents thereof.

7. Use according to claim 5 or 6 wherein said virus lacks both a functional gene encoding ICP27 and a functional gene encoding ICP4.

8. Use according to any one of claims 4 to 7 wherein said virus lacks both a functional gene encoding ICP27 and a functional gene encoding ICP4 and which has a VMW65 gene, or a functional equivalent thereof, which encodes a protein which lacks transcriptional-activation activity.

9. Use according to any one of claims 5 to 8 wherein virus lacks functional genes encoding ICPO, ICP4, ICP22 and ICP27.

10. Use according to any one of the preceding claims wherein said virus further lacks a functional ICP34.5 gene, or a functional equivalent thereof.

11. Use according to any one of the preceding claims wherein said virus comprises a heterologous gene.

12. Use according to claim 11 wherein said heterologous gene is operably linked to a control sequence permitting expression of said heterologous gene in a dendritic cell.

13. Use according to claim 11 or 12 wherein said heterologous gene encodes a tumour antigen.

14. Use according to claim 13, wherein said tumour antigen is increased in or on the surface of tumour cells as compared to non-tumour cells.

15. Use according to claim 13, wherein said tumour antigen is present in or on the surface of tumour cells but is absent from non-tumour cells.

16. Use according to claim 11 or 12 wherein said heterologous gene encodes a polypeptide capable of modulating an immune response.

17. Use according to claim 16 wherein said polypeptide is chemokine, cytokine or co-stimulatory molecule.

18. Use according to claim 11 or 12 wherein said heterologous gene encodes a polypeptide of parasitic, viral or bacterial origin.

19. Use according to claim 18, wherein said polypeptide is selected from hepatitis C virus antigens, hepatitis B surface or core antigens, papillomavirus antigens, HIV antigens and malaria antigens.

20. Use according to any one core of claims 11 to 19 wherein said virus comprises more than one heterologous gene.

21. Use according to any one of the preceding claims wherein said dendritic cells are human dendritic cells.

22. Use according to any one of the preceding claims wherein said dendritic cells are isolated or prepared from peripheral blood or bone marrow prior to infection *ex vivo*.

23. Use according to claim 22 wherein said dendritic cells are returned to the body after infection.

24. Use according to any one of claims 1 to 21 wherein the said dendritic cells are infected *in vivo* following administration of the virus to the human or animal body.

25. Use according to any one of the preceding claims wherein the medicament is for administration by injection, by infusion, by an intra- or trans-dermal route or by biolistic means.

26. Use according to any one of the preceding claims wherein the medicament is for treatment or prevention of a pathogenic infection or cancer.

27. Use of *ex vivo* dendritic cells infected with an attenuated herpes virus as defined in any one of claims 1 to 16 in the manufacture of a medicament for use in a method of stimulating an immune response.

## Patentansprüche

1. Verwendung eines abgeschwächten Herpes-Virus, welchem/s
(i) ein funktionelles vhs-Gen, oder ein funktionelles Äquivalent davon, fehlt;
(ii) ein funktionelles UL43-Gen, oder ein funktionelles Äquivalent davon, umfasst;
in der Herstellung eines Medikaments zur Verwendung bei einer Methode zur Stimulierung einer Immunantwort, welche Methode das Infizieren von dendritischen Zellen mit dem Virus umfasst.

2. Verwendung nach Anspruch 1, wobei das Virus ein Herpes-simplex-Virus 1 oder 2 ist.

3. Verwendung nach Anspruch 1 oder 2, wobei dem Virus ein für ICP47 codierendes funktionelles Gen fehlt.

4. Verwendung nach einem der vorangehenden Ansprüche, wobei das Virus ein VMW65-Gen, oder ein funktionelles Äquivalent davon, aufweist, welches für ein Protein codiert, dem eine Aktivität für die transkriptionelle Aktivierung fehlt.

5. Verwendung nach einem der vorangehenden Ansprüche, wobei dem Virus mindestens ein funktionelles immediate early-Gen fehlt.

6. Verwendung nach Anspruch 5, wobei das immediate early-Gen ausgewählt ist aus Genen, die für ICP0, ICP4, lCP22, ICP27 oder funktionelle Äquivalente davon codieren.

7. Verwendung nach Anspruch 5 oder 6, wobei dem Virus sowohl ein für ICP27 codierendes funktionelles Gen als auch ein für ICP4 codierendes funktionelles Gen fehlt.

8. Verwendung nach einem der Ansprüche 4 bis 7, wobei dem Virus sowohl ein für ICP27 codierendes funktionelles Gen als auch ein für ICP4 codierendes funktionelles Gen fehlt, und welches ein VMW65-Gen, oder ein funktionelles Äquivalent davon, aufweist, das für ein Protein codiert, dem eine Aktivität für die transkriptionelle Aktivierung fehlt.

9. Verwendung nach einem der Ansprüche 5 bis 8, wobei dem Virus die für ICP0, ICP4, ICP22 und ICP27 codierenden funktionellen Gene fehlen.

10. Verwendung nach einem der vorangehenden Ansprüche, wobei dem Virus außerdem ein funktionelles ICP34,5-Gen, oder ein funktionelles Äquivalent davon, fehlt.

11. Verwendung nach einem der vorangehenden Ansprüche, wobei das Virus ein heterologes Gen umfasst.

12. Verwendung nach Anspruch 11, wobei das heterologe Gen funktionsfähig an eine Kontrollsequenz geknüpft ist, welche die Expression des heterologen Gens in einer dendritischen Zelle erlaubt.

13. Verwendung nach Anspruch 11 oder 12, wobei das heterologe Gen für ein Tumorantigen codiert.

14. Verwendung nach Anspruch 13, wobei das Tumorantigen in oder auf der Oberfläche von Tumorzellen erhöht vorkommt im Vergleich zu nicht-Tumorzellen.

15. Verwendung nach Anspruch 13, wobei das Tumor-Antigen in oder auf der Oberfläche von Tumorzellen vorhanden ist, doch in nicht-Tumorzellen abwesend ist.

16. Verwendung nach Anspruch 11 oder 12, wobei das heterologe Gen für ein Polypeptid codiert, das zum Modulieren einer Immunantwort fähig ist.

17. Verwendung nach Anspruch 16, wobei das Polypeptid Chemokin, Cytokin oder costimulatorisches Molekül ist.

18. Verwendung nach Anspruch 11 oder 12, wobei das heterologe Gen für ein Polypeptid von parasitärem, viralem oder bakteriellem Ursprung codiert.

19. Verwendung nach Anspruch 18, wobei das Polypeptid ausgewählt ist aus Hepatitis-C-Virus-Antigenen, Hepatitis-B-Oberflächen- oder -Kern-Antigenen, Papillomavirus-Antigenen, HIV-Antigenen und Malaria-Antigenen.

20. Verwendung nach einem der Ansprüche 11 bis 19, wobei das Virus mehr als ein heterologes Gen umfasst.

21. Verwendung nach einem der vorangehenden Ansprüche, wobei die dendritischen Zellen humane dendritische Zellen sind.

22. Verwendung nach einem der vorangehenden Ansprüche, wobei die dendritischen Zellen aus peripherem Blut oder Knochenmark vor der *ex vivo*infektion isoliert oder präpariert werden.

23. Verwendung nach Anspruch 22, wobei die dendritischen Zellen dem Körper nach Infektion zurückgegeben werden.

24. Verwendung nach einem der Ansprüche 1 bis 21, wobei die dendritischen Zellen auf die Verabreichung des Virus an den menschlichen oder tierischen Körper hin *in vivo* infiziert werden.

25. Verwendung nach einem der vorangehenden Ansprüche, wobei das Medikament zur Verabreichung durch Injektion, durch Infusion, auf einem intra- oder transdermalen Weg oder durch biolistische Methoden dient.

26. Verwendung nach einem der vorangehenden Ansprüche, wobei das Medikament zur Behandlung oder Verhütung einer pathogenen Infektion oder von Krebs ist.

27. Verwendung von ex vivo mit einem abgeschwächten Herpes-Virus infizierten dendritischen Zellen, wie in einem der Ansprüche 1 bis 16 definiert, in der Herstellung eines Medikaments zur Verwendung bei einer Methode zur Stimulierung einer Immunantwort.

## Revendications

1. Utilisation d'un herpèsvirus atténué qui
(i) est dépourvu de gène vhs fonctionnel ou d'un équivalent fonctionnel de celui-ci ;
(ii) comprend un gène UL43 fonctionnel ou l'un de ses équivalents fonctionnels ;
dans la fabrication d'un médicament destiné à être utilisé dans un procédé de stimulation d'une réponse immunitaire, ce procédé comprenant l'infection de cellules dendritiques avec ledit virus.

2. Utilisation selon la revendication 1, dans laquelle ledit virus est un virus herpès simplex 1 ou 2.

3. Utilisation selon la revendication 1 ou 2, dans laquelle ledit virus est dépourvu d'un gène fonctionnel codant ICP47.

4. Utilisation selon l'une quelconque des revendications précédentes, dans laquelle ledit virus a un gène VMW65, ou l'un de ses équivalents fonctionnels, qui code une protéine qui est dépourvue d'activité d'activation de la transcription.

5. Utilisation selon l'une quelconque des revendications précédentes, dans laquelle ledit virus est dépourvu d'au moins un gène précoce immédiat fonctionnel.

6. Utilisation selon la revendication 5, dans laquelle ledit gène précoce immédiat est choisi parmi les gènes codant ICPO, ICP4, ICP22, ICP27 ou leurs équivalents fonctionnels.

7. Utilisation selon la revendication 5 ou 6, dans laquelle ledit virus est dépourvu d'un gène fonctionnel codant ICP27 et d'un gène fonctionnel codant ICP4.

8. Utilisation selon l'une quelconques des revendications 4 à 7, dans laquelle ledit virus est dépourvu d'un gène fonctionnel codant ICP27 et d'un gène fonctionnel codant ICP4, et a un gène VMW65, ou l'un de ses équivalents fonctionnels, qui code une protéine qui est dépourvue d'activité d'activation de la transcription.

9. Utilisation selon l'une quelconque des revendications 5 à 8, dans laquelle le virus est dépourvu de gènes fonctionnels codant ICPO, ICP4, ICP22 et ICP27.

10. Utilisation selon l'une quelconque des revendications précédentes, dans laquelle ledit virus est en outre dépourvu d'un gène ICP34.5 fonctionnel ou de l'un de ses équivalents fonctionnels.

11. Utilisation selon l'une quelconque des revendications précédentes, dans laquelle ledit virus comprend un gène hétérologue.

12. Utilisation selon la revendication 11, dans laquelle ledit gène hétérologue est lié de façon opérationnelle à une séquence de contrôle permettant l'expression dudit gène hétérologue dans une cellule dendritique.

13. Utilisation selon la revendication 11 ou 12, dans laquelle ledit gène hétérologue code un antigène tumoral.

14. Utilisation selon la revendication 13, dans laquelle ledit antigène tumoral est présent en quantités plus élevées dans ou sur la surface de cellules tumorales que dans ou sur celle de cellules non tumorales.

15. Utilisation selon la revendication 13, dans laquelle ledit antigène tumoral est présent dans ou sur la surface des cellules tumorales, mais est absent des cellules non tumorales.

16. Utilisation selon la revendication 11 ou 12, dans laquelle ledit gène hétérologue code un polypeptide capable de moduler une réponse immunitaire.

17. Utilisation selon la revendication 16, dans laquelle ledit polypeptide est une chimiokine, une cytokine ou une molécule co-stimulatrice.

18. Utilisation selon la revendication 11 ou 12, dans laquelle ledit gène hétérologue code un polypeptide d'origine parasitaire, viral ou bactérienne.

19. Utilisation selon la revendication 18, dans laquelle ledit polypeptide est choisi parmi des antigènes de virus d'hépatite C, des antigènes de surface ou de nucléocapside d'hépatite B, des antigènes de papillomavirus, des antigènes de VIH et des antigènes de malaria.

20. Utilisation selon l'une quelconque des revendications 11 à 19, dans laquelle ledit virus comprend plus d'un gène hétérologue.

21. Utilisation selon l'une quelconque des revendications précédentes, dans laquelle lesdites cellules dendritiques sont des cellules dendritiques humaines.

22. Utilisation selon l'une quelconque des revendications précédentes, dans laquelle lesdites cellules dendritiques sont isolées ou préparées à partir de sang périphérique ou de moelle osseuse avant infection *ex vivo.*

23. Utilisation selon la revendication 22, dans laquelle lesdites cellules dendritiques sont réintroduites dans l'organisme après infection.

24. Utilisation selon l'une quelconque des revendications 1 à 21, dans laquelle lesdites cellules dendritiques sont infectées *in vivo* après administration du virus à l'organisme humain ou animal.

25. Utilisation selon l'une quelconque des revendications précédentes, dans laquelle le médicament est destiné à une administration par injection, par perfusion, par voie intra-ou trans-dermique, ou par des moyens biolistiques.

26. Utilisation selon l'une quelconque des revendications précédentes, dans laquelle le médicament est destiné au traitement ou à la prévention d'une infection par un pathogène ou d'un cancer.

27. Utilisation de cellules dendritiques *ex vivo* infectées par un herpesvirus atténué, telle que définies dans l'une quelconques des revendications 1 à 16, dans la fabrication d'un médicament destiné à être utilisé dans un procédé de stimulation d'une réponse immunitaire.
